Europäisches Patentamt

European Patent Office

Office européen des brevets

(11) Publication number: **0 402 776**
**A2**

# EUROPEAN PATENT APPLICATION

(21) Application number: **90110836.5**

(22) Date of filing: **07.06.90**

(51) Int. Cl.⁵: **A61K 7/48**

(30) Priority: **08.06.89 JP 147965/89**

(43) Date of publication of application:
**19.12.90 Bulletin 90/51**

(84) Designated Contracting States:
**DE FR GB IT**

(71) Applicant: **Sansho Seiyaku Co., Ltd.**
**26-7, Oike 2-chome**
**Onojo-shi Fukuoka-ken(JP)**

(72) Inventor: **Oyama, Yasuaki**
**15-16 Oike 2-chome**
**Onojo-shi, Fukuoka-ken(JP)**

(74) Representative: **Patentanwälte Deufel- Schön-**
**Hertel- Lewald**
**Isartorplatz 6**
**D-8000 München 2(DE)**

(54) **Melanogenesis-inhibiting preparation for external application.**

(57) Disclosed is a melanogenesis-inhibiting preparation for external application, which contains one or more selected from N-acyl-O-acylmannosamines and N-acyl-O-acylgalactosamines as an active ingredient. The preparation is of use for the remedy of or prevention of chromatosis. It is applied to the face in the form of a cream, milky lotion, lotion, creamy pack, ointment and emulsion.

EP 0 402 776 A2

# MELANOGENESIS-INHIBITING PREPARATION FOR EXTERNAL APPLICATION

## BACKGROUND OF THE INVENTION

### Field of the Invention

The present invention relates to a melanogenesis-inhibiting preparation for external application, which contains one or more selected from N-acyl-O-acylmannosamines and N-acyl-O-acylgalactosamines as an active ingredient and which is effective for the remedy and prevention of chromatosis such as chloasma (liver spots) and for the whitening of a human skin.

### Prior Art

For a long time, a cosmetic material containing a peroxide such as hydrogen peroxide or zinc peroxide has been used for the purpose of removing blotches such as spots or freckles appeared on the skin. However, the peroxides are extremely unstable and therefore problems arise on their storage stability. Additionally, stable incorporation of such a peroxide into a cosmetic base is difficult and the peroxides themselves would not have a sufficient whitening effect. On the other hand, a cosmetic material containing vitamin C, cystein or colloidal sulfur has become utilized for the purpose of skin-whitening. However, the effect of such substances is still unsatisfactory.

Additionally, a kojic acid-containing skin-whitening cosmetic material (Japanese Patent Publication No. 56-18569), a kojic acid-containing melanogenesis-inhibiting ointment (Japanese Patent Publication No. 61-10447), and a kojic acid derivative-containing skinwhitening cosmetic material (Japanese Patent Publication Nos. 61-60801 and 61-60802, Japanese Patent Unexamined Publication No. 56-79616) are disclosed. Further, skin-whitening cosmetic materials containing quercetin or a flavonol compound (Japanese Patent Unexamined Publication Nos. 55-92305, 55-111410, 55-111411 and 55-143908) are also disclosed.

In addition, a skin-whitening cosmetic material containing an extract from placentae (Japanese Patent Publication No. 48-30370), a melanogenesis-inhibiting preparation for external application containing vitamin E and kojic acid (Japanese Patent Unexamined Publication No. 62-178506), and a skin-whitening cosmetic material containing an aqueous solution of vitamin E (Japanese Patent Unexamined Publication No. 56-75421) are also illustrated.

On the other hand, a cosmetic material containing an amino sugar, an N-acetylamino sugar, or an N-alkylamino sugar alcohol for the purpose of improving the soft touch to the skin, the moisturizing effect, the softening effect and the skin-activating effect (Japanese Patent Unexamined Publication Nos. 59-13708 and 59-212419) and a skin-whitening cosmetic material containing glucosamine or an acylated glucosamine derivative (Japanese Patent Unexamined Publication No. 62-36306) are disclosed.

Among the components as used in the known skin-whitening agents mentioned above, glutathione, cystein and vitamin C are unstable and have an extremely insufficient melanogenesis-inhibiting action on living cells.

On the other hand, kojic acid, flavonol compounds and vitamin E have a melanogenesis-inhibiting action on living cells and are useful substances that display a skin-whitening effect. However, the method of forming a preparation containing such a substance is difficult.

Under the situation, the present inventors studied the melanogenesis-inhibiting action of mannosamine derivatives and galactosamine derivatives and repeatedly investigated the access of the said derivatives to cells: for example, mouse melanoma-derived B16 cells. As a result, they have found that N-acyl-O-acyl derivatives of the said substances have a remarkable melanogenesis-inhibiting effect against B16 cells. On the basis of such finding, they have accomplished the present invention which is effective for the remedy of chromatosis such as chloasma and for the whitening of spots or freckles. .

## SUMMARY OF THE INVENTION

The present invention provides a melanogenesis-inhibiting preparation for external application, which contains one or more selected from N-acyl-O-acylmanosamines and N-acyl-O-acylgalactosamines as an active ingredient.

## DETAILED DESCRIPTION OF THE INVENTION

N-acyl-O-acylmanosamines and N-acyl-O-acylgalactosamines which are employed in the present invention as an active ingredient are prepared by acylating the amino group and the four hydroxyl groups of mannosamine or galactosamine, and the acyl group in the compounds is preferably to be derived from a fatty acid having from 2 to 21 carbon atoms.

Specifically, N-acyl-O-acylmannosamines include N-acetyl-1,3,4,6-tetra-O-acetylmannosamine, N-acetyl-1,3,4,6-tetra-O-propionylmannosamine, N-propionyl-O-1,3,4,6-tetra-propionylmannosamine, N-acetyl-O-1,3,4,6-tetra-O-octynylmannosamine and N-acetyl-O-tetranonadecanylmannosamine; and N-acyl-O-acylgalactoamines include N-acetyl-1,3,4,6-tetra-O-acetylgalactosamine, N-acetyl-1,3,4,6-tetra-O-propionylgalactosamine, N-propionyl-1,3,4,6-tetra-O-acetylgalactosamine, N-propionyl-1,3,4,6-tetra-O-propionyl-galatosamine, N-acetyl-1,3,4,6-tetra-O-octynylgalactosamine and N-acetyl-1,3,4,6-tetra-O-nonadecanylgalactosamine.

The preparation for eternal application of the present invention is prepared by a known method, using one or more selected from N-acyl-O-acylmannosamines and N-acyl-O-acylgalactosamines as the active ingredient along with bases or agents which are generally employed in preparing ordinary cosmetic materials such as emulsion, lotion, liniment, ointment, toilet water, cream, or milky lotion.

The content of the active ingredient in the preparation of the present invention is from 0.001 to 20% by weight, preferably from 0.01 to 10% by weight, to the total weight of the preparation.

Next, a test example to support the melanogenesis-inhibiting activity of the preparation of the present invention is mentioned below.

## TEST EXAMPLE

Each of N-acetyl-1,3,4,6-tetra-O-acetylmannosamine, N-acetyl-1,3,4,6-tetra-O-acetylgalactosamine, N-acetyl-1,3,4,6-tetra-O-propionylmannosamine, N propionyl-1,3,4,6-tetra-O-propionylgalactosamine, N-acetyl-1,3,4,6-tetra-O-octynylmannosamine and N-acetyl-1,3,4,6-tetra-O-nonadecanylgalactosamine was added to a liquid medium containing mouse melanoma-derived B16 cells (hereinafter referred to as "B16 cells") each in a concentration of 0.005, 0.010 or 0.020 mg/ml, and the cells were incubated at 37°C for 5 days. After incubation, the number of the cells was counted. Next, the blackened degree (degree of melanine formation) of each cell pellet was observed by naked eye.

The test results are shown in Table 1 below.

| Compound Tested | Concentration (mg/ml) | Whiteness of Cells | Number of Cells/51 cm$^2$ dish |
|---|---|---|---|
| N-acetyl-1,3,4,6-tetra-O-acetylmannosamine | 0.005<br>0.010<br>0.020 | +<br>+ +<br>+ + + | 3.0×10<br>2.5×10<br>2.0×10 |
| N-acetyl-1,3,4,6-tetra-O-acetylgalactosamine | 0.005<br>0.010<br>0.020 | +<br>+ +<br>+ + + | 3.0×10<br>2.5×10<br>2.0×10 |
| N-acetyl-1,3,4,6-tetra-O-propionylmannosamine | 0.005<br>0.010<br>0.020 | +<br>+ +<br>+ + + | 2.8×10<br>2.1×10<br>1.7×10 |
| N-propionyl-1,3,4,6-tetra-O-propionylgalactosamine | 0.005<br>0.010<br>0.020 | ±<br>+<br>+ + | 2.5×10<br>2.0×10<br>1.6×10 |
| N-acetyl-1,3,4,6-tetra-O-octynylmannosamine | 0.005<br>0.010<br>0.020 | ±<br>+<br>+ + | 2.1×10<br>1.7×10<br>1.1×10 |
| N-acetyl-1,3,4,6-tetra-O-nonadecanylgalactosamine | 0.005<br>0.010<br>0.020 | ±<br>+<br>+ + | 2.1×10<br>1.5×10<br>1.0×10 |
| Control (No compound added) | | - | 3.1×10 |

Whiteness of Cells:

(-) Black
(±) Gray
( + ) Grayish white
( + + ) White

As is obvious from the test results shown above, N-acetyl-1,3,4,6-tetra-O-acetylmannosamine, N-acetyl-1,3,4,6-tetra-O-acetylgalactosamine and N-acetyl-1,3,4,6-tetra-O-propionylmannosamine almost completely whitened the cells, each in a concentration of 0.010 mg/ml. N-propionyl-1,3,4,6-tetra-O-propionylgalactosamine, N-acetyl-1,3,4,6-tetra-O-octynylmannosamine and N-acetyl-1,3,4,6-tetra-O-nonadecanylgalactosamine almost completely whitened the cells, each in a concentration of 0.020 mg/ml. Accordingly, the active ingredients of the present invention have an excellent melanogenesis-inhibiting activity.

Next, examples of the present inventions are mentioned below.

EXAMPLE 1 (Cream)

4

| Ingredients (% by weight): | |
|---|---|
| 1. Polyethylene Glycol Monostearate (40E.O.) | 2.00 |
| 2. Self-emulsifying Glycerin Monostearate | 5.00 |
| 3. Stearic Acid | 5.00 |
| 4. Behenyl Alcohol | 1.00 |
| 5. Liquid Paraffin | 10.00 |
| 6. Glycerin Trioctanoate | 10.00 |
| 7. N-acetyl-1,3,4,6-tetra-O-acetylmannosamine | 0.50 |
| 8. Parahydroxybenzoate | 0.20 |
| 9. 1,3-Butylene Glycol | 5.00 |
| 10. Disodium Edetate | 0.01 |
| 11. Pure Water | 61.29 |

Manufacturing Method:

(A) (1) to (7) are heated and dissolved.
(B) (8) to (11) are heated and dissolved.
(C) (B) is added to (A), emulsified, stirred and cooled.
(D) (C) is cooled and filled in a container.
After tested, it is used as a product.

Directions and Dosage:

A proper amount of the cream is applied and rubbed on the face.

EXAMPLE 2 (Milky Lotion)

| Ingredients (% by weight): | |
|---|---|
| 1. Polyoxyethylene Sorbitan Monostearate (20E.O.) | 1.00 |
| 2. Polyoxyethylene Sorbitol Tetraoleate (60E.O.) | 0.50 |
| 3. Oleophilic Glycerin monostearate | 1.00 |
| 4. Stearic Acid | 0.50 |
| 5. Behenyl Alcohol | 0.50 |
| 6. Avocado Oil | 4.00 |
| 7. Glyceryl Trioctanoate | 4.00 |
| 8. N-acetyl-1,3,4,6-tetra-O-acetylgalactosamine | 0.10 |
| 9. Parahydroxybenzoate | 0.20 |
| 10. 1,3-Butylene Glycol | 5.00 |
| 11. Xanthane Gum | 0.14 |
| 12. Disodium Edetate | 0.10 |
| 13. Pure Water | 83.05 |

Manufacturing Method:

(A) (1) to (8) are heated and dissolved.
(B) (9) to (13) are heated and dissolved.
(C) (B) is added to (A), emulsified, stirred and cooled.
(D) (C) is cooled and tilled in a container.

After tested, it is used as a product.

Directions and Dosage:

A proper amount of the milky lotion is applied and rubbed on the face.

EXAMPLE 3 (Lotion)

| Ingredients (% by weight): | |
|---|---|
| 1. Polyoxyethylene-Hardened Castor Oil (60E.O.) | 8.00 |
| 2. Ethanol | 15.00 |
| 3. N-acetyl-1,3,4,6-tetra-O-acetylgalactosamine | 0.10 |
| 4. Parahydroxybenzoate | 0.10 |
| 5. Citric Acid | 0.10 |
| 6. Sodium Citrate | 0.30 |
| 7. 1,3-Butylene Glycol | 4.00 |
| 8. Disodium Edetate | 0.01 |
| 9. Pure Water | 72.39 |

Manufacturing Method:

(A) (1) to (9) are uniformly stirred and dissolved.
(B) (A) is filled in a container, which is tested and then used as a product.

Directions and Dosage:

A proper amount of the lotion is applied and rubbed on the face.

EXAMPLE 4 (Creamy Pack)

| Ingredients (% by weight): | |
|---|---|
| 1. Polyethylene Glycol Monostearate (40E.O.) | 2.00 |
| 2. Self-emulsifying Glycerin Monostearate | 5.00 |
| 3. Stearic Acid | 5.00 |
| 4. Behenyl Alcohol | 0.50 |
| 5. Squalane | 15.00 |
| 6. Cetyl Octanoate | 5.00 |
| 7. N-acetyl-1,3,4,6-tetra-O-propionylmannosamine | 1.00 |
| 8. Parahydroxybenzoate | 0.20 |
| 9. 1,3-Butylene Glycol | 5.00 |
| 10. Disodium Edetate | 0.01 |
| 11. Pure Water | 61.29 |

Manufacturing Method:

(A) (1) to (7) are heated and dissolved.

6

(B) (8) to (11) are heated and dissolved.
(C) (B) is added to (A), emulsified, stirred and cooled.
(D) (C) is cooled and filled in a container.
After tested, it is used as a product.

Directions and Dosage:

A proper amount of the creamy pack is applied and rubbed on the face.

EXAMPLE 5 (Ointment)

| Ingredients (% by weight): | |
|---|---|
| 1. Polyethylene Glycol Monostearate (40E.O.) | 2.00 |
| 2. Self-emulsifying Glycerin Monostearate | 5.00 |
| 3. Stearic Acid | 5.00 |
| 4. Behenyl Alcohol | 1.00 |
| 5. Liquid Paraffin | 10.00 |
| 6. Glyceryl Trioctanoate | 10.00 |
| 7. N-acetyl-1,3,4,6-tetra-O-propionylmannosamine | 1.00 |
| 8. Parahydroxybenzoate | 0.20 |
| 9. 1,3-Butylene Glycol | 5.00 |
| 10. Disodium Edetate | 0.01 |
| 11. Pure Water | 60.79 |

Manufacturing Method:

(A) (1) to (7) are heated and dissolved.
(B) (8) to (11) are heated and dissolved.
(C) (B) is added to (A), emulsified, stirred and cooled.
(D) (C) is cooled and filled in a container.
After tested, it is used as a product.

Directions and Dosage:

A proper amount of the ointment is applied and rubbed on the face.

EXAMPLE 6 (Emulsion)

7

| Ingredients (% by weight): | |
|---|---|
| 1. Polyoxyethylene Sorbitan Monostearate (20E.O.) | 1.00 |
| 2. Polyoxyethylene Sorbitol Tetraoleate (60E.O.) | 0.50 |
| 3. Oleophilic Glycerin Monostearate | 1.00 |
| 4. Stearic Acid | 0.50 |
| 5. Behenyl Alcohol | 0.50 |
| 6. Avocado Oil | 4.00 |
| 7. Glyceryl Trioctanoate | 4.00 |
| 8. N-acetyl-1,3,4,6-tetra-O-propionylmannosamine | 1.00 |
| 9. Parahydroxybenzoate | 0.20 |
| 10. 1,3-Butylene Glycol | 5.00 |
| 11. Xanthane Gum | 0.14 |
| 12. Disodium Edetate | 0.01 |
| 13. Pure Water | 82.15 |

Manufacturing Method:

    (A) (1) to (8) are heated and dissolved.
    (B) A part of (9) to (13) is heated and dissolved.
    (C) (B) is added to (A), emulsified, stirred and cooled.
    (D) (C) is cooled and filled in a container.
After tested, it is used as a product.

Direction and Dosage:

A proper amount of the lotion is applied and rubbed on the face.

As mentioned above, the active ingredients of the present invention are almost free from toxicity to cells and have an excellent melanogenesis-inhibiting activity. The present invention therefore provides an excellent melanogenesis-inhibiting preparation for external application.

While the invention has been described in detail and with reference to specific embodiments thereof, it will be apparent to one skilled in the art that various changes and modifications can be made therein without departing from the spirit and scope thereof.

**Claims**

1. A melanogenesis-inhibiting preparation for external application, which contains one or more selected from N-acetyl-1,3,4,6-tetra-O-acylmannosamines and N-acetyl-1,3,4,6-tetra-O-acylgalactosamines as an active ingredient.

2. The melanogenesis-inhibiting preparation for external application as claimed in claim 1, in which the N-acetyl-1,3,4,6-tetra-O-acylmannosamines are N-acetyl-1,3,4,6-tetra-O-acetylmannosamine, N-acetyl-1,3,4,6-tetra-O-propionylmannosamine, N-propionyl-O-1,3,4,6-tetra-propionylmannosamine, N-acetyl-O-1,3,4,6-tetra-O-octynylmannosamine, and N-acetyl-O-tetranonadecanylmannosamine.

3. The melanogenesis-inhibiting preparation for external application as claimed in claim 1, in which the N-acetyl-O-acyl-galactosamines are N-acetyl-1,3,4,6-tetra-O-acetylgalactosamine, N-acetyl-1,3,4,6-tetra-O-propionylgalactosamine, N-propionyl-1,3,4,6-tetra-O-acetylgalactosamine, N-propionyl-1,3,4,6-tetra-O-propionylgalactosamine, N-acetyl-1,3,4,6-tetra-O-octynylgalactosamine, and N-acetyl-1,3,4,6-tetra-O-nonadecanylgalactosamine.

4. The melanogenesis-inhibiting preparation for external application as claimed in any one of claims 1 to 3, in which the content of the active ingredient is from 0.001 to 20% by weight to the total weight of the preparation.

5. The melanogenesis-inhibiting preparation for external application as claimed in claim 4, in which the content of the active ingredient is from 0.01 to 10% by weight to the total weight of the preparation.

6. The melanogenesis-inhibiting preparation for external application as claimed in any one of claims 1 to 5, which is in the form of cream, milky lotion, lotion, creamy pack, ointment and emulsion to be applied to the face.